## Europäisches Patentamt

## European Patent Office

## Office européen des brevets

(19)

(11) Veröffentlichungsnummer: **0 191 464**
**A2**

(12) **EUROPÄISCHE PATENTANMELDUNG**

(21) Anmeldenummer: **86101742.4**

(22) Anmeldetag: **12.02.86**

(51) Int. Cl.⁴: **C 07 H 9/04**

(30) Priorität: **15.02.85 DE 3505150**

(43) Veröffentlichungstag der Anmeldung: **20.08.86**
Patentblatt 86/34

(84) Benannte Vertragsstaaten: **CH DE FR GB LI NL**

(71) Anmelder: **BASF Aktiengesellschaft,
Carl-Bosch-Strasse 38, D-6700 Ludwigshafen (DE)**

(72) Erfinder: **Pfaff, Klaus-Peter, Dr., Lindenhofstrasse 76a,
D-6800 Mannheim 1 (DE)**
Erfinder: **Paust, Joachim, Dr., Ringstrasse 3,
D-6708 Neuhofen (DE)**
Erfinder: **Hartmann, Horst, Lindenstrasse 45,
D-6737 Boehl-Iggelheim (DE)**

(54) **Verfahren zur Herstellung von Zuckerketalen.**

(57) Verfahren zur Herstellung von Zuckerketalen durch Umsetzen von Zuckern mit Ketonen in Gegenwart von einem sauren Katalysator, das dadurch gekennzeichnet ist, dass man

A. eine Bortrifluorid-Molekülverbindung oder Trifluormethansulfonsäure in Mengen von nur 0,01 bis 10 Gew.-%, bezogen auf eingesetzten Zucker, verwendet,

B. das Keton in bis zu 30fach molarem Überschuss einsetzt und

C. das bei der Reaktion gebildete Wasser laufend aus dem Reaktionsgemisch entfernt.

Besonders vorteilhaft gestaltet sich das Verfahren, wenn man das Bortrifluorid als Bortrifluorid-Etherat einsetzt, dieses bzw. die Trifluormethansulfonsäure nach Beendigung der Umsetzung unter nichtwässrigen Bedingungen unwirksam macht und anschliessend das Reaktionsgemisch nach Abdampfen des Ketons einer fraktionierten Destillation unterwirft. Besondere Bedeutung hat das Verfahren für die Umsetzung von L-Sorbose mit Aceton zu der für die Synthese von Ascorbinsäure benötigten 2,3:4,6-Di-O-isopropyliden-L-sorbofuranose.

EP 0 191 464 A2

## Verfahren zur Herstellung von Zuckerketalen

Die vorliegende Erfindung betrifft ein Verfahren zur Herstellung von Zuckerketalen, bei dem der Säurekatalysator in katalytischen Mengen eingesetzt wird.

Zuckerketale sind häufig synthetisierte Zuckerderivate, wenn es um den Schutz der Hydroxylgruppen des Zuckers geht. So ist beispielsweise die 2,3:4,6-Di-O-isopropyliden-$\alpha$-L-sorbose (Diaceton-L-Sorbose) ein wichtiges Zwischenprodukt bei der Herstellung von Vitamin C nach dem sogenannten "Reichsteinverfahren".

Zur Herstellung von Zuckerketalen sind sehr viele verschiedene Methoden beschrieben worden. Zur Durchführung der Reaktion unter thermodynamischer Kontrolle zwischen dem betreffenden Zucker und dem Keton eignen sich verschiedene Säurekatalysatoren. Es sind dies zum einen Mineralsäuren wie Schwefel-, Jod- und Bromwasserstoff-, Salz-, Phosphor- oder Perchlorsäure. Zum anderen sind es saure Ionenaustauscher oder organische Säuren wie Essig- und Sulfonsäuren. Darüber hinaus können auch Lewissäuren oder Schwermetallsalze verwendet werden. Hierzu gehören Zinn- und Eisenchlorid, Kupfersulfat und weitere Kupfersalze sowie die Chloride seltener Erden.

Zur Erzielung hoher Umsätze ist es erforderlich, das während der Reaktion gebildete Wasser zu binden oder zu entfernen. Aus diesem Grund wird beispielsweise Schwefelsäure häufig verwendet. Der Nachteil dabei ist, daß große Mengen an Säure eingesetzt werden müssen, die am Ende der Reaktion mit Alkali zu neutralisieren sind, wodurch entsprechende Mengen an Sulfaten anfallen.

Zur Vereinfachung der Aufarbeitung und Entsorgung sind daher auch schon Methoden ausgearbeitet worden, bei denen nur katalytische Mengen an Säure eingesetzt werden.

So wird in der DE-OS 20 03 067 ein Verfahren zur Herstellung von Zuckern beschrieben, bei dem katalytische Mengen an Perchlorsäure, Eisen(III)-chlorid oder Eisen(III)bromid als Katalysator verwendet werden. Das gebildete Reaktionswasser wird dabei azeotrop-destillativ entfernt. In den europäischen Patentanmeldungen 76 118 und 91 223 wird die Verwendung von Kupfersalzen bzw. Iodwasserstoffsäure beschrieben. Kritisch bei diesen Verfahren sind teilweise die Ausbeuten sowie die Reinheiten der zu synthetisierenden Zuckerketale. Nur wenn die Stärke und die Menge des verwendeten Säurekatalysators optimal gewählt werden, kann die Bildung von störenden Nebenprodukten unterdrückt werden. Speziell für die Herstellung

Rr/HB

der als Zwischenprodukt für die Vitamin-C-Synthese benötigten 2,3:4,6-Di-O-isopropyliden-α-L-sorbofuranose ergeben sich bei den bekannten Verfahren beachtliche Nachteile. So können beispielsweise bei Verwendung von Schwermetallen als Katalysatoren in den darauffolgenden Reaktionsstufen der Vitamin-C-Synthese durch enthaltene Katalysatorspuren Probleme auftreten.

Auch die Verwendung von Bortrifluorid-Etherat als Katalysator für die Umsetzung von Zuckern mit Aldehyden und Ketonen in einem geeigneten Lösungsmittel, wie Dimethylsulfoxid oder Dioxan, ist schon untersucht worden (vgl. E. Bergonzi et al., "Die Stärke" 16 (1964), Seiten 386-90 und C. Boffi et al., ibid 21 (1969), S. 100). Hierbei wurden nur bei der Umsetzung mit Aldehyden gute Ausbeuten an Zuckeracetalen erhalten. Weit weniger geeignet scheint nach diesen Veröffentlichungen die Methode für die Umsetzung von Zuckern mit Ketonen zu sein. Dies gilt besonders für die Umsetzung in Dimethylsulfoxid. Doch auch bei der Umsetzung von D-Glucose mit Aceton in Dioxan als Lösungsmittel wurde trotz Verwendung von 2 Äquivalenten BF₃-Etherat, bezogen auf den eingesetzten Zucker, das gewünschte Ketal nur in Ausbeuten von 20 % erhalten. Außerdem ist die aufwendige Entfernung der Lösungsmittel ein entscheidender Nachteil dieses Verfahrens. Bei der Umsetzung von Methylglycosiden mit Formaldehyd (aus 1,3,5-Trioxan) in Dioxan als Lösungsmittel und BF₃-Etherat als Katalysator wurden die entsprechenden 4,6-O-Methylenverbindungen nur in einer Ausbeute von weniger als 30 % erhalten (vgl. J.C. Goodwin et al., "Carbohydr. Res. 28 (1973), Seiten 213-19).

Es war daher die Aufgabe der Erfindung, das Verfahren zur Herstellung von Zuckerketalen, insbesondere für die Herstellung der für die Vitamin-C-Synthese wichtigen Diaceton-L-sorbose, so zu verbessern, daß auch ohne den Einsatz von Schwermetallkatalysatoren die Umsetzung von Zuckern mit Ketonen auf einfache Weise in Gegenwart von nur katalytischen Mengen eines sauren Katalysators mit guten Ausbeuten und guter Reinheit gelingt und dabei die Aufarbeitung des Reaktionsgemisches sich möglichst einfach gestaltet.

Es wurde nun überraschenderweise gefunden, daß man auch bei der Umsetzung von Zuckern mit geeigneten Ketonen in Gegenwart von Bortrifluorid-diethyletherat oder Trifluormethansulfonsäure als saurem Katalysator gute Ausbeuten von Zuckerketalen erhält, wenn man die Umsetzung in Gegenwart von nur katalytischen Mengen, d.h. Mengen von nur 0,01 bis 10 Gew.%, bezogen auf eingesetzten Zucker, an Bortrifluorid-diethyletherat oder Trifluormethansulfonsäure vornimmt und das bei der Reaktion gebildete Wasser laufend aus dem Reaktionsgemisch entfernt. Überraschend war auch, daß unter diesen Bedingungen, insbesondere bei der Umsetzung mit Aceton, auf die Mitverwendung eines die Löslichkeit des Zuckers erhöhenden Lösungsmittels, wie Dioxan oder Dimethylsulfoxid, verzichtet werden kann. Durch die Verwendung von nur katalytischen Mengen an der $BF_3$-Verbindung oder Trifluormethansulfonsäure und Verzicht auf ein zusätzliches Lösungsmittel ließ sich die anschließende Aufarbeitung des Reaktionsgemisches wesentlich einfacher durchführen.

Gegenstand der Erfindung ist daher ein Verfahren zur Herstellung von Zuckerketalen durch Umsetzen von Zuckern mit Ketonen in Gegenwart von einem saurem Katalysator, das dadurch gekennzeichnet ist, daß man

A. die Bortrifluorid-Molekülverbindung oder Trifluormethansulfonsäure in Mengen von nur 0,01 bis 10 Gew.%. bezogen auf eingesetzten Zucker verwendet,

B. das Keton in bis zu 30fach molarem Überschuß einsetzt und

C. das bei der Reaktion gebildete Wasser laufend aus dem Reaktionsgemisch entfernt.

Gegenstand der Erfindung ist insbesondere ein Verfahren zur Herstellung von Diaceton-L-sorbose durch Umsetzen von L-Sorbose mit Aceton in Gegenwart von einem sauren Katalysator, das dadurch gekennzeichnet ist, daß man

A. Bortrifluorid-Diethyletherat oder Trifluormethansulfonsäure in Mengen von nur 0,01 bis 10 Gew.%. bezogen auf eingesetzten Zucker, verwendet,

B. das Keton in bis zu 30fach molarem Überschuß einsetzt und

C. das bei der Reaktion gebildete Wasser laufend aus dem Reaktionsgemisch entfernt.

Besonders vorteilhaft gestaltet sich das Verfahren, wenn man das Bortrifluorid, die Trifluormethansulfonsäure oder allgemein katalytische Mengen eines geeigneten sauren Katalysators nach Beendigung der Umsetzung unter nichtwäßrigen Bedingungen unwirksam macht und anschließend nach Abdestillieren des Ketons das Reaktionsgemisch direkt durch fraktionierte Destillation aufarbeitet. Besondere Bedeutung hat das Verfahren für die Umsetzung von L-Sorbose mit Aceton zu der für die Synthese von Ascorbinsäure benötigten 2,3:4,6-Di-O-isopropyliden-L-sorbofuranose.

Es ist zwar aus der EP-OS 138 436 schon ein Verfahren zur Ketalisierung von 2-Ketogulonsäure bekannt, bei dem als saure Ketalisierungskatalysatoren unter sehr vielen anderen auch Bortrifluorid-Diethyletherat und Tri-

BASF Aktiengesellschaft — 4 — 0 191 464

fluormethansulfonsäure genannt werden. Jedoch ist eine $\alpha$-Ketozuckersäure nicht mit Ribose vergleichbar - und außerdem werden in gleichzeitig oder früher angemeldeten Erfindungen des gleichen Arbeitskreises über die Ketalisierung von Zuckern (vgl. EP 139 486, EP 76 118 oder EP 91 223) nur ganz spezielle Ketalisierungskatalysatoren, wie $SbCl_5$, $SbF_5$, $HJ$, $CuCl_2$ oder $CuBr_2$ auch in nur katalytischen Mengen als brauchbar genannt.

Folgende Zucker können mit Hilfe des erfindungsgemäßen Verfahrens ketalisiert werden:

Pentosen wie Ribose, Arabinose, Xylose, Lyxose, Ribulose oder Xylulose; Hexosen wie Glucose, Mannose, Gulose, Idose, Galactose, Fructose oder Sorbose; Deoxyzucker wie Rhamnose, 2-Deoxyglucose oder 2-Deoxyribose und Zuckeralkohole wie Ribitol, Mannitol oder Sorbitol.

Als Ketone kommen für das erfindungsgemäße Verfahren acyclische Ketone, wie Aceton, Methylethylketon, Diethylketon, Dipropylketone und Dibutylketone sowie cyclische Ketone, wie Cyclopentanon, Cyclohexanon und Cycloheptanon in Betracht. Von besonderer Bedeutung ist die Ketalisierung mit Aceton, die mit sehr guten Ausbeuten gelingt. Ketalisierungen mit cyclischen Ketonen, wie Cyclohexanon, verlaufen ganz allgemein mit weniger guten Ausbeuten.

Die einzusetzende Menge an Keton ist unterschiedlich und hängt von der betreffenden Verbindung ab. Es muß auch berücksichtigt werden, ob ein Mono-, ein Di- oder ein Triketal gebildet werden kann und soll. In der Regel wird das Keton in großem molarem Überschuß verwendet, d.h. von mehr als 10fach molarer Menge bis etwa 30fach molarer Menge, vorzugsweise etwa 20 bis 25fach molarer Menge. Dabei dient das Keton als Reaktant und gleichzeitig als Lösungsmittel. Auf die Mitverwendung eines die Löslichkeit des Zuckers erhöhenden Lösungsmittels, wie Dioxan oder Dimethylsulfoxid kann im allgemeinen verzichtet werden, wodurch sich die Aufarbeitung des Reaktionsgemisches wesentlich vereinfacht und dadurch verbilligt. Bei Verwendung von höhersiedenden Ketonen, wie dem bei 156°C siedenden Cyclohexanon, kann es jedoch vorteilhaft sein, ein Lösungsmittel mitzuverwenden, welches mit diesem Keton ein niedriger siedendes Azeotrop bildet, um Überhitzung im Reaktionsgemisch zu vermeiden. Genannt seien als solche Lösungsmittel beispielsweise Methylenchlorid und Dimethoxyethan.

Bortrifluorid wird als Katalysator in Form einer Molekülverbindung mit Ethern, Carbonsäuren, Dialkylsulfiden oder Fluorwasserstoff eingesetzt, vorzugsweise als Bortrifluorid-Diethyletherat.

Die Menge des als Katalysator einzusetzenden Bortrifluorid-Etherats bzw. der Trifluormethansulfonsäure liegt im katalytischen Bereich. Die Menge sollte zwischen 0,1 und 10 Gew.%, vorzugsweise zwischen 1 und 5 Gew.%, bezogen auf das Gewicht des als Ausgangsmaterial verwendeten Zuckers, liegen. Bei Verwendung von größeren Mengen an Bortrifluorid sind die erzielten Ausbeuten überraschender Weise wesentlich geringer.

Zur Durchführung des erfindungsgemäßen Verfahrens wird eine Mischung bestehend aus dem zu ketalisierenden Zucker, dem Keton und dem $BF_3$-Komplex bzw. der Trifluormethansulfonsäure unter Rückfluß zum Sieden erhitzt. Die Reaktionstemperatur beträgt je nach Art der eingesetzten Reaktanten und dem gewählten Druck zwischen etwa 20 und 150°C bei Normaldruck oder reduziertem Druck.

Zur Entfernung des bei der Reaktion gebildeten Wassers aus dem Reaktionsgemisch sind verschiedene Methoden bekannt. So kann beispielsweise das unter Rückflußbedingungen gebildete, im wesentlichen aus dem Keton und dem Reaktionswasser bestehende Kondensat vor dessen Rückfluß in das Umsetzungsgefäß getrocknet werden. Dies kann beispielsweise sehr vorteilhaft dadurch erfolgen, daß man zwischen den Rückflußkühler und das Umsetzungsgefäß einen mit einem Molekularsieb gefüllten Soxhlet-Extraktor schaltet. Man kann aber auch das Wasser enthaltende Keton abdestillieren und das abdestillierte Keton durch trockenes ersetzen.

Man kann aber auch das Wasser durch azeotropes Abdestillieren mit einem inerten mit Wasser ein Azeotrop bildendem Lösungsmittel entfernen. Dazu eignen sich besonders solche Lösungsmittel, die niedriger sieden als das umzusetzende Keton. Für die Umsetzung mit Aceton sind das z.B. Lösungsmittel mit einem Siedepunkt unterhalb von 56°C, vorzugsweise 30 bis 50°C, wie Methylenchlorid, Pentan oder Cyclopentan. Das niedrigsiedende Lösungsmittel verwendet man mit Vorteil nur in geringeren Mengen (etwa 50 bis 100 ml pro 500 ml Keton), damit es sich am Anfang der Umsetzung im wesentlichen nur oben in der Kolonne befindet und nicht im eigentlichen Umsetzungsgefäß, damit es nicht die ohnehin schlechte Löslichkeit des Zuckers in dem Keton noch weiter herabsetzt. Erst wenn die feste Sorbose in Lösung gegangen ist, stört ein größerer Anteil an dem Azeotrop bildenden inerten Lösungsmittel im Umsetzungsgefäß nicht mehr.

Die Reaktionszeiten hängen von der Art des zu ketalisierenden Zuckers, der Katalysatormenge und den übrigen Reaktionsbedingungen ab. Sie liegen zwischen etwa 5 Minuten und 10 Stunden, vorzugsweise zwischen etwa 1 Stunde und 8 Stunden.

Zur Isolierung der Zuckerketale kühlt man das Reaktionsgemisch im allgemeinen auf Temperaturen zwischen etwa 20 und -30°C ab und macht den sauren Katalysator beispielsweise durch Zugabe einer wäßrigen NaHCO$_3$-Lösung unwirksam, extrahiert die wäßrige Phase mehrfach mit einem geeigneten Extraktionsmittel, wie Benzol oder Toluol, welches nach dem Waschen und Trocknen des Extrakts destillativ von dem gebildeten Zuckerketal entfernt wird.

Besonders vorteilhaft gestaltet sich das erfindungsgemäße Verfahren, wenn man das Bortrifluorid bzw. die Trifluormethansulfonsäure nach Beendigung der Umsetzung unter nichtwäßrigen Bedingungen unwirksam macht und anschließend das Reaktionsgemisch nach Abdampfen des Ketons einer fraktionierten Destillation unterwirft. Dies ist z.B. möglich durch Zugabe stöchiometrischer Mengen eines Alkalialkoholats, insbesondere Natriummethylat oder Kaliummethylat oder durch Umsetzen mit entsprechenden Mengen Ammoniak, Aminen oder anderen basischen Verbindungen bzw. Alkalioder Erdalkalihalogeniden, wie NaF, LiF oder CaF$_2$. Das Reaktionsgemisch kann dann - gegebenenfalls nach Filtration -unter vermindertem Druck eingeengt und nach Abdampfen des Ketons einer fraktionierten Destillation unterworfen werden.

Nach einer besonders bevorzugten Ausführungsform des erfindungsgemäßen Verfahrens arbeitet man das Reaktionsgemisch so auf, daß man das Bortrifluorid bzw. die Trifluormethansulfonsäure durch Zugabe etwa äquimolarer Mengen an Natrium- oder Kaliummethylat unwirksam macht und anschließend das Reaktionsgemisch destillativ aufarbeitet, d.h. zunächst das überschüssige Keton und gegebenenfalls das Lösungsmittel aus dem Reaktionsgemisch abdestilliert und dann das erhaltene Rohprodukt einer fraktionierten Destillation unterwirft. Dies hat den - besonderen -Vorteil, daß die in technischem Maßstab aufwendige Aufarbeitung durch Extraktion, Waschung und Eindampfung gänzlich entfällt und das gewünschte Produkt in guten Ausbeuten und vor allem in hoher Reinheit erhalten wird.

Es hat sich gezeigt, daß sich die großen Vorteile, die sich dadurch ergeben, daß man bei der Aufarbeitung des Reaktionsgemisches das Bortrifluorid unter nichtwäßrigen Bedingungen, insbesondere durch Zugabe stöchiometrischer Mengen eines Alkalialkoholats, unwirksam macht und anschließend das Reaktionsgemisch nach Abdampfen des Ketons einer fraktionierten Destillation unterwirft, auch dann ergeben, wenn man die Ketalisierung der Zucker in Gegenwart von geeigneten Protonensäuren als sauren Katalysatoren vornimmt, sofern diese in nur katalytischen Mengen, also etwa in Mengen von 0,01 bis etwa 10 Gew.%, vorzugsweise etwa 1 bis 5 Gew.%, bezogen auf den eingesetzten Zucker, verwendet werden. Als ge-

eignete Protonensäuren seien beispielsweise $H_2SO_4$, HJ und organische
Sulfonsäuren, wie p-Toluolsulfonsäure, genannt.

Gegenstand der Erfindung ist daher auch ein Verfahren zur Herstellung von
Zuckerketalen durch Umsetzen von Zuckern mit Ketonen in Gegenwart von
0,01 bis 10 Gew.%, vorzugsweise 1 bis 5 Gew.%, bezogen auf eingesetzten
Zucker, einer geeigneten Protonensäure als saurem Katalysator unter ständigem Entfernen des bei der Reaktion gebildeten Wassers, das dadurch gekennzeichnet ist, daß man die Protonensäure nach Beendigung der Umsetzung
durch Zugabe stöchiometrischer Mengen eines Alkalialkoholats unter nichtwäßrigen Bedingungen unwirksam macht und anschließend das Reaktionsgemisch nach Abdampfen des Ketons einer fraktionierten Destillation unterwirft.

Mit Hilfe des erfindungsgemäßen Verfahrens können Zuckerketale, insbesondere die für die Synthese von Ascorbinsäure benötigte 2,3:4,6-Di-0-isopropyliden-L-sorbofuranose auf besonders vorteilhafte Weise hergestellt
werden.

Beispiel 1

Eine Mischung aus 15 g (833 mmol) wasserfreier D-Glucose, 0,38 ml
(3,1 mmol) Bortrifluorid-Diethyletherat und 333 ml (4,54 mol) Aceton
($H_2O$-Gehalt <0,1 %) wurde 8 Stunden (h) unter Rückfluß zum Sieden erhitzt. Dabei befanden sich 40 g Molekularsieb (3 Å, Fa. Grace GmbH) in
einem Soxhlet-Extraktor zwischen Kolben und Rückflußkühler. Nach Beendigung der Reaktion wurde das Reaktionsgemisch auf $-10^0$C abgekühlt und mit
10 ml verd. wäßriger Natriumhydrogencarbonatlösung versetzt. Anschließend
wurde das Aceton unter vermindertem Druck abdestilliert, der Rückstand
mit 50 ml Wasser aufgenommen und 3 x mit 50 ml Toluol extrahiert. Die
vereinigten Toluolphasen wurden mit wenig Wasser gewaschen und über
Natriumsulfat getrocknet. Nach Abdestillieren des Toluols unter vermindertem Druck wurden 19,9 g (92 %) 1,2:5,6-Di-0-isopropyliden-α-D-gluco-
furanose erhalten. Nach Umkristallisation aus Hexan/Aceton (15:1) wurde
ein Schmelzpunkt von $110-112^0$C bestimmt.

Beispiel 2

Eine Mischung aus 15 g (833 mmol) D-Mannose, 0,38 ml Bortrifluorid-
Etherat und 333 ml Aceton ($H_2O$-Gehalt <0,1 %) wurde 2,5 h unter Rückfluß
zum Sieden erhitzt, wobei das Kondensat - wie in Beispiel 1 beschrieben -
getrocknet wurde. Nach Aufarbeitung analog Beispiel 1 wurden 17,0 g

BASF Aktiengesellschaft - 8 - 0 191 464

(78 %) 2,3:5,6-Di-O-isopropyliden-α-D-mannofuranose erhalten, welches nach Umkristallisation aus n-Hexan einen Schmelzpunkt von 122-124⁰C aufwies.

Beispiel 3

Eine Mischung aus 20 g (111 mmol) D-Galactose, 0,5 ml (4,1 mmol) Bortrifluorid-Etherat und 400 ml (5,45 mol) Aceton wurde 5,5 h unter Rückfluß zum Sieden erhitzt, wobei das Kondensat wie in Beispiel 1 beschrieben getrocknet wurde. Nach Aufarbeitung analog Beispiel 1 wurden 25,9 g (90 %) 1,2:3,4-Di-O-isopropyliden-α-D-galactopyranose erhalten. Nach Chromatographie an Kieselgel (660, Fa. Merck) mit Hexan/Aceton (6:4) wurde ein öliges Produkt erhalten.

Elementaranalyse ($C_{12}H_{20}O_6$):
ber. (%)   C 55,4   H 7,7
gef. (%)      55,5      7,8

Beispiel 4

Eine Mischung aus 15 g (833 mmol) L-Sorbose, 0,38 ml Bortrifluorid-Etherat und 333 ml Aceton wurde 6 h unter Rückfluß zum Sieden erhitzt. Dabei befanden sich 40 g Molekularsieb (3 Å) in einem Soxhlet-Extraktor zwischen Kolben und Rückflußkühler. Nach Beendigung der Reaktion wurde das Reaktionsgemisch auf -10⁰C abgekühlt und mit 10 ml wäßriger Natriumhydrogencarbonatlösung versetzt. Anschließend wurde das Aceton unter vermindertem Druck abdestilliert, der Rückstand in 200 ml Toluol aufgenommen und 2mal mit 30 ml 5 %iger wäßriger NaHCO₃-Lösung extrahiert. Anschließend wurde mit 10 ml verd. NaCl-Lösung gewaschen und über Natriumsulfat getrocknet. Nach Abdestillieren des Toluols unter vermindertem Druck wurden 18,7 g (86 %) 2,3:4,6-Di-O-isopropyliden-α-L-sorbofuranose erhalten. Nach Umkristallisation aus Hexan wurde ein Schmelzpunkt von 78-79,5⁰C gemessen.

Beispiel 5

Eine Mischung aus 90 g L-Sorbose, 2,28 ml Bortrifluorid-Etherat und 2 Litern Aceton wurde 6 h unter Rückfluß zum Sieden erhitzt, wobei sich 240 g Molekularsieb (3 Å, Fa. Grace GmbH) in einem Soxhlet-Extraktor zwischen Reaktionsgefäß und Rückflußkühler befanden. Nach Beendigung der Reaktion wurde das Reaktionsgemisch auf 0⁰C abgekühlt und mit 1,47 g Natriummethylat versetzt. Drei entsprechende Reaktionsansätze wurden vereinigt und hieraus das Aceton unter vermindertem Druck abdestilliert. Es wurden 393 g Rückstand erhalten, der in einer Füllkörperkolonne bis 0,4 bar fraktioniert destilliert wurde. Dabei wurden 268,1 g (68,8 %) 2,3:4,6-Di-0-isopropyliden-α-L-sorbofuranose mit einer Reinheit von 96 bis 98 %, sowie 35,0 g (9,0 %) Produkt mit einer Reinheit von 92 % erhalten. 16,1 g (4,1 %) Produkt verblieben in der Kolonne, die bei Rückführungen mitverwendet werden könnten.

Beispiel 6

Eine Mischung aus 12 g (666 mmol) Sorbose, 0,5 ml (4,1 mmol) Bortrifluorid-Etherat und 400 ml Aceton wurde 3 h unter Rückflußkühlung und Trocknung des rückfließenden Kondensats analog Beispiel 5 zum Sieden erhitzt. Nach Aufarbeitung analog Beispiel 4 wurden 14,1 g (81 %) 2,3:4,6-Di-0-isopropyliden-α-L-sorbofuranose erhalten.

Beispiel 7

Eine Mischung aus 20 g (111 mmol) Sorbose, 2 ml (16,4 mmol) Bortrifluorid-Etherat und 400 ml Aceton wurde 4 h unter Rückfluß zum Sieden erhitzt, wobei das rückfließende Kondensat analog Beispiel 1 getrocknet wurde. Nach Zugabe von 0,4 g (10,5 mmol) Natriummethylat und Aufarbeitung analog Beispiel 5 wurden 23,5 g (81 %) 2,3:4,6-Di-0-isopropyliden-α-L-sorbofuranose erhalten.

Beispiel 8

Eine Mischung aus 20 g (133 mmol) D-Arabinose, 0,5 ml (4,1 mmol) Bortrifluorid-Etherat und 400 ml Aceton wurde 2 h unter Rückfluß zum Sieden erhitzt, wobei das rückfließende Kondensat analog Beispiel 1 getrocknet wurde. Nach Aufarbeitung analog Beispiel 1 wurden 27,4 g (89 %) 1,2:3,4-Di-0-isopropyliden-β-D-arabinopyranose (Reinheit 98 %) erhalten. Nach Kristallisation aus n-Hexan wurde ein Schmelzpunkt von 41-41,5°C gemessen.

Beispiel 9

Eine Mischung aus 20 g D-Xylose, 0,5 ml Bortrifluorid-Etherat und 400 ml Aceton wurde 4 h unter Rückfluß zum Sieden erhitzt, wobei das rückfließende Kondensat analog Beispiel 1 getrocknet wurde. Nach Aufarbeitung analog Beispiel 1 wurden 28,4 g (93 %) 1,2:3,5-Di-0-isopropyliden-α-D-xylofuranose (Reinheit 96 %) erhalten. Nach Umkristallisation aus n-Hexan wurde ein Schmelzpunkt von 43-45°C gemessen.

Beispiel 10

Eine Mischung aus 10 g (66,6 mmol) D-Ribose, 0,25 ml Bortrifluorid-Etherat und 200 ml Aceton wurde 5 min unter Rückfluß zum Sieden erhitzt, wobei das rückfließende Kondensat analog Beispiel 1 getrocknet wurde. Nach Beendigung der Reaktion wurden 5 ml wäßrige Natriumhydrogencarbonatlösung zugegeben und das Aceton unter vermindertem Druck abdestilliert. Der Rückstand wurde anschließend an Kieselgel (G 60, Fa. Merck) mit Hexan/Aceton (3:2) chromatographisch gereinigt. Erhalten wurden 7,8 g (62 %) 2,3-Isopropyliden-D-ribofuranose.

Elementaranalyse ($C_8H_{14}O_5$)
ber. (%)  C 50,5   H 7,4
gef. (%)     50,6      7,5

Beispiel 11

Eine Mischung aus 20 g D-Mannit, 0,5 ml Bortrifluorid-Etherat und 400 ml Aceton wurde 4 h unter Rückfluß zum Sieden erhitzt, wobei das rückfließende Kondensat analog Beispiel 1 getrocknet wurde. Nach Aufarbeitung analog Beispiel 1 wurden 31,5 g (95 %) 1,2:3,4:5,6-Tri-O-isopropyliden-D-mannit (Reinheit 97 %) erhalten. Nach Umkristallisation aus n-Hexan wurde ein Schmelzpunkt von 69-70°C gemessen.

Beispiel 12

Eine Mischung aus 20 g D-Fructose, 0,5 ml Bortrifluorid-Etherat und 400 ml Aceton wurde 3,5 h unter Rückfluß zum Sieden erhitzt, wobei das rückfließende Kondensat analog Beispiel 1 getrocknet wurde. Nach Durchführung der Reaktion und Aufarbeitung wie in Beispiel 1 wurden 26,3 g (91 %) 2,3:4,6-Di-O-isopropyliden-$\beta$-D-fructopyranose erhalten. Nach Umkristallisation aus n-Hexan wurde ein Schmelzpunkt von 96-98°C gemessen.

Beispiel 13

Eine Mischung aus 5 g D-Arabinose, 0,13 ml Bortrifluorid-Diethyletherat, 75 ml Cyclohexanon und 60 ml Dichlormethan wurde 90 Minuten unter Rückfluß zum Sieden erhitzt. Dabei befanden sich 20 g Molekularsieb in einem Soxhlet-Extraktor zwischen Kolben und Rückflußkühler. Nach Beendigung der Reaktion wurde das Reaktionsgemisch auf +20°C abgekühlt, mit 150 ml Toluol verdünnt, mit wäßriger $NaHCO_3$-Lösung extrahiert, mit Wasser gewaschen und über $Na_2SO_4$ getrocknet. Nach Abdestillieren des Lösungs-

BASF Aktiengesellschaft - 11 - **0 191 464**

mittelgemisches unter vermindertem Druck wurden 9,8 g (95 %) 1,2:3,4-Di-O-cyclohexyliden-$\beta$-D-arabinopyranose erhalten. Nach Umkristallisation aus n-Hexan wurde ein Schmelzpunkt von 71,5 bis 73°C gemessen.

Beispiel 14

Eine Mischung aus 10 g wasserfreier D-Glucose, 0,25 ml Bortrifluorid-Diethyletherat, 150 ml Dimethoxyethan und 50 ml Cyclohexanon wurde 80 Minuten unter Rückfluß zum Sieden erhitzt. Nach Durchführung der Reaktion und Aufarbeitung analog Beispiel 13 und Umkristallisation aus n-Hexan wurden 7,5 g (40 %) 1,2:5,6-Di-O-cyclohexyliden-$\alpha$-D-glucofuranose mit einem Schmelzpunkt von 133,5 bis 135°C erhalten.

Beispiel 15

Eine Mischung aus 20 g L-Sorbose, 0,5 ml Bortrifluorid-Diethyletherat, 100 ml Cyclohexanon und 300 ml Dimethoxyethan wurde 25 Minuten unter Rückfluß zum Sieden erhitzt. Nach Durchführung der Reaktion und Aufarbeitung analog Beispiel 13 und Umkristallisation aus n-Hexan wurden 16,6 g (44 %) 2,3:4,6-Di-O-cyclohexyliden-$\alpha$-L-sorbofuranose mit einem Schmelzpunkt von 122 bis 123,5°C erhalten.

Beispiel 16

In einer Apparatur, bestehend aus einem Dreihalskolben, Füllkörperkolonne, Wasserauskreiser und Rückflußkühler wurde eine Mischung aus 20 g L-Sorbose, 400 ml Aceton und 0,5 ml Bortrifluorid-Diethyletherat unter Rückfluß zum Sieden erhitzt. Über den Wasserauskreiser wurden 70 ml n-Pentan zugesetzt. Diese Menge war so bemessen, daß sich das n-Pentan nur im Dampf-, nicht aber im Reaktionsraum befand. Nach 3 h wurden weitere 50 ml n-Pentan zugesetzt und bei einer Sumpftemperatur von 50°C wurde noch 12 h unter Rückfluß erhitzt. Am Wasserabscheider wurden dabei 5,8 ml Unterphase mit 62,5 Gew.% Wasser erhalten.

Nach Beendigung der Reaktion wurde das Reaktionsgemisch auf 10°C abgekühlt und mit 0,33 g Natriummethylat versetzt. Anschließend wurde das Aceton unter vermindertem Druck abdestilliert, der Rückstand in 100 ml Toluol aufgenommen, 2 x mit 10 ml 5 %iger $NaHCO_3$-Lösung extrahiert und mit 5 ml 5 %iger NaCl-Lösung gewaschen. Nach Trocknen über $Na_2SO_4$ und Abdestillieren des Toluols unter vermindertem Druck wurden 24,7 g (85,5 %) 2,3:4,6-Di-O-iosopropyliden-$\alpha$-L-sorbofuranose erhalten.

Beispiel 17

Eine Mischung aus 20 g (111 mmol) L-Sorbose, 200 μl (2,3 mmol) Trifluormethansulfonsäure (TFMSA) und 400 ml Aceton (H$_2$O-Gehalt 0,1 %) wurde
4,5 Stunden (h) unter Rückfluß zum Sieden erhitzt. Dabei befanden sich
40 g Molekularsieb (3 Å) in einem Soxhlet-Extraktor zwischen Reaktionskolben und Rückflußkühler. Nach Beendigung der Reaktion wurde das Reaktionsgemisch auf -10°C abgekühlt und mit 10 ml wäßriger NaHCO$_3$-Lösung
versetzt. Anschließend wurde das Aceton unter vermindertem Druck abdestilliert, der Rückstand in 35 ml H$_2$O aufgenommen und 3mal mit je 110 ml
Toluol bei 70°C extrahiert. Die vereinigten Toluolphasen wurden mit
7,5 ml H$_2$O gewaschen und über Na$_2$SO$_4$ getrocknet. Nach Abdestillieren des
Toluols unter vermindertem Druck wurden 24,7 g (85,5 %) 2,3:4,6-Di-0-
isopropyliden-α-L-sorbofuranose (DAS) erhalten. Nach Umkristallisation
aus Toluol wurde ein Schmelzpunkt von 78 bis 79°C gemessen.

Beispiel 18

Eine Mischung aus 20 g (111 mmol) L-Sorbose, 100 μl (1,1 mmol) TFMSA und
400 ml Aceton wurde 2,5 h unter Rückfluß zum Sieden erhitzt unter Trocknung des rückfließenden Kondensats analog Beispiel 1. Nach Abkühlung,
Neutralisation und Abdestillation des Acetons wurde der Rückstand in
300 ml Toluol aufgenommen und bei Raumtemperatur mit 15 ml 5 %iger wäßriger NaHCO$_3$-Lösung und 15 ml H$_2$O extrahiert. Nach Trocknen über Na$_2$SO$_4$
und Abdestillieren des Toluols wurden 24,8 g (85,7 %) DAS erhalten.

Beispiel 19

Eine Mischung aus 20 g (111 mmol) L-Sorbose, 10 μl (0,11 mmol) TFMSA und
400 ml Aceton wurde 7 h analog Beispiel 1 zum Sieden erhitzt. Nach Abkühlung des Reaktionsgemisches auf -10°C wurde 1 ml konzentrierte wäßrige
NH$_4$OH-Lösung zugesetzt. Nach Filtration wurde das Aceton abdestilliert
und der Rückstand in 300 ml Toluol aufgenommen. Es wurde analog Beispiel 2 weiter aufgearbeitet. Dabei wurden 24,1 g (83,3 %) DAS erhalten.

Beispiel 20

In einer Apparatur, bestehend aus einem Dreihalskolben, Füllkörperkolonne, Wasserauskreiser und Rückflußkühler wurde eine Mischung aus 30 g
L-Sorbose, 600 ml Aceton und 15 μl TFMSA unter Rückfluß zum Sieden erhitzt. Über den Wasserauskreiser wurden 75 ml Cyclopentan zugesetzt. Nach
6 h wurden weitere 100 ml Cyclopentan zugegeben und es wurde noch 18 h
unter Rückfluß erhitzt. Am Wasserabscheider wurden insgesamt 10,5 ml Un-

terphase mit 51 Gew.% $H_2O$ erhalten. Nach Beendigung der Reaktion wurde das Reaktionsgemisch auf $0^0C$ abgekühlt und mit 500 µl 5 %iger $NaOCH_3$/ $CH_3OH$-Lösung versetzt. Nach dem Eindampfen, Aufnehmen in 450 ml Toluol und Auswaschen mit 15 ml $NaHCO_3$-Lösung sowie 10 ml $H_2O$, Trocknen und Eindampfen wurden 40,3 g (93,0 %) DAS erhalten.

Beispiel 21

Eine Mischung aus 90 g L-Sorbose, 45 µl TFMSA und 1800 ml Aceton wurde 12 h unter Rückfluß zum Sieden erhitzt, wobei 180 g Molekularsieb zum Trocknen des Kondensats verwendet wurden. Nach Beendigung der Reaktion wurde das Reaktionsgemisch auf $0^0C$ abgekühlt und mit 1 ml 5 %iger $NaOCH_3$/ $CH_3OH$-Lösung versetzt. Die Reaktionsprodukte von vier entsprechenden Reaktionsansätzen wurden vereinigt und daraus das Aceton abdestilliert. Es wurden 535 g Rückstand erhalten, von dem 470 g in einer Füllkörperkolonne bei 0,1 bis 0,25 mbar fraktioniert destilliert wurden. Dabei wurden 306 g (67,0 %) DAS mit einer Reinheit von 96 bis 97,5 %, 45 g (9,9 %) mit einer Reinheit von 94 % erhalten, 30,5 g (6,7 %) verblieben in der Säule.

Patentansprüche

1. Verfahren zur Herstellung von Zuckerketalen durch Umsetzen von Zuckern mit Ketonen in Gegenwart von einem sauren Katalysator, dadurch gekennzeichnet, daß man

   A.  eine Bortrifluorid-Molekülverbindung oder Trifluormethansulfonsäure in Mengen von nur 0,01 bis 10 Gew.%, bezogen auf eingesetzten Zucker, verwendet,

   B.  das Keton in bis zu 30fach molarem Überschuß einsetzt und

   C.  das bei der Reaktion gebildete Wasser laufend aus dem Reaktionsgemisch entfernt.

2. Verfahren zur Herstellung von Diaceton-L-sorbose durch Umsetzen von L-Sorbose mit Aceton in Gegenwart von einem sauren Katalysator, dadurch gekennzeichnet, daß man

   A.  Bortrifluorid-Diethyletherat oder Trifluormethansulfonsäure in Mengen von nur 0,01 bis 10 Gew.%, bezogen auf eingesetzten Zucker, verwendet,

   B.  das Keton in bis zu 30fach molarem Überschuß einsetzt und

   C.  das bei der Reaktion gebildete Wasser laufend aus dem Reaktionsgemisch entfernt.

3. Verfahren zur Herstellung von Zuckerketalen gemäß Anspruch 1 durch Umsetzen von Zuckern mit Ketonen in Gegenwart von Bortrifluorid in Form einer Molekülverbindung als saurem Katalysator, dadurch gekennzeichnet, daß man Bortrifluorid-Diethyletherat in Mengen von nur 0,01 bis 10 Gew.%, bezogen auf eingesetzten Zucker, verwendet,

4. Verfahren zur Herstellung von Zuckerketalen gemäß Anspruch 3, dadurch gekennzeichnet, daß man als Zucker eine Pentose, eine Hexose, einen Deoxyzucker oder einen Zuckeralkohol verwendet.

5. Verfahren zur Herstellung von Zuckerketalen gemäß Anspruch 1 und 3, dadurch gekennzeichnet, daß man als Zucker L-Sorbose verwendet.

6. Verfahren zur Herstellung von Zuckerketalen gemäß Anspruch 1 und 3, dadurch gekennzeichnet, daß man als Keton Aceton verwendet.

7. Verfahren zur Herstellung von Zuckerketalen gemäß Anspruch 5 und 6, dadurch gekennzeichnet, daß man auf die Mitverwendung eines die Löslichkeit des Zuckers erhöhenden Lösungsmittels verzichtet.

8. Verfahren zur Herstellung von Zuckerketalen gemäß Anspruch 1 und 3, <u>dadurch gekennzeichnet</u>, daß man das Wasser aus dem Reaktionsgemisch durch azeotropes Abdestillieren mit Hilfe eines inerten Lösungsmittels mit einem Siedepunkt unterhalb von 56°C entfernt.

9. Verfahren zur Herstellung von Zuckerketalen gemäß Anspruch 1 und 2, <u>dadurch gekennzeichnet</u>, daß man das Wasser aus dem unter Rückfluß siedenden Reaktionsgemisch durch Trocknen des im wesentlichen aus dem Keton und Wasser bestehenden Kondensats vor dessen Rückfluß in das Umsetzungsgefäß entfernt.

10. Verfahren zur Herstellung von Zuckerketalen gemäß Anspruch 1, <u>dadurch gekennzeichnet</u>, daß man das Bortrifluorid bzw. die Trifluormethansulfonsäure nach Beendigung der Umsetzung durch Zugabe etwa äquimolarer Mengen an Natriummethylat unwirksam macht und anschließend das Reaktionsgemisch nach Abdampfen des Ketons einer fraktionierten Destillation unterwirft.

11. Verfahren zur Herstellung von Zuckerketalen gemäß Anspruch 3, <u>dadurch gekennzeichnet</u>, daß man

   A. das Bortrifluorid in Form eines Bortrifluorid-Diethyletherats verwendet,
   B. als Keton Aceton einsetzt und auf die Mitverwendung eines die Löslichkeit des Zuckers erhöhenden Lösungsmittels verzichtet und
   C. das bei der Reaktion gebildete Wasser durch azeotropes Abdestillieren mit Hilfe eines inerten Lösungsmittels mit einem Siedepunkt unterhalb von 56°C laufend aus dem Reaktionsgemisch entfernt.

12. Verfahren zur Herstellung von Diaceton-L-sorbose durch Umsetzen von L-Sorbose mit Aceton gemäß Anspruch 1, <u>dadurch gekennzeichnet</u>, daß man

   A. Trifluormethansulfonsäure in Mengen von 0,01 bis 10 Gew.%, bezogen auf die eingesetzte Sorbose, als sauren Katalysator verwendet,
   B. das Aceton in bis zu 30fach molarem Überschuß einsetzt und
   C. das bei der Reaktion gebildete Wasser laufend aus dem Reaktionsgemisch entfernt.

13. Verfahren zur Herstellung von Diaceton-L-sorbose durch Umsetzen von L-Sorbose mit Aceton gemäß Anspruch 12, dadurch gekennzeichnet, daß man auf die Mitverwendung eines die Löslichkeit des Zuckers erhöhenden Lösungsmittels verzichtet und das bei der Reaktion gebildete Wasser durch azeotropes Abdestillieren mit Hilfe eines inerten Lösungsmittels mit einem Siedepunkt unterhalb von 56°C laufend aus dem Reaktionsgemisch entfernt.

14. Verfahren zur Herstellung von Diaceton-L-sorbose durch Umsetzen von L-Sorbose mit Aceton gemäß Anspruch 12, dadurch gekennzeichnet, daß man die Trifluormethansulfonsäure nach Beendigung der Umsetzung durch Zugabe etwa äquimolarer Mengen an Natriummethylat unwirksam macht und anschließend das Reaktionsgemisch nach Abdampfen des Ketons und des inerten Lösungsmittels einer fraktionierten Destillation unterwirft.

15. Verfahren zur Herstellung von Zuckerketalen durch Umsetzen von Zuckern mit Ketonen in Gegenwart von 0,01 bis 10 Gew.%, vorzugsweise 1 bis 5 Gew.%, bezogen auf eingesetzten Zucker, einer geeigneten Protonensäure als sauren Katalysator unter ständigem Entfernen des bei der Reaktion gebildeten Wassers, dadurch gekennzeichnet, daß man die Protonensäure nach Beendigung der Umsetzung durch Zugabe stöchiometrischer Mengen eines Alkalialkoholats unter nicht-wäßrigen Bedingungen unwirksam macht und anschließend das Reaktionsgemisch nach Abdampfen des Ketons einer fraktionierten Destillation unterwirft.